# FASCICULE DE BREVET EUROPEEN

(11) **EP 3 875 579 B1**
(45) Date de publication et mention de la délivrance du brevet: **16.07.2025**
(21) Numéro de dépôt: 20305215.4
(22) Date de dépôt: 02.03.2020
(51) Int. Cl.: C12N 5/071

(54) **PROCEDE D'OBTENTION DE CELLULES ENDOTHELIALES A PARTIR DE CELLULES SOUCHES PLURIPOTENTES**
VERFAHREN ZUR ERZEUGUNG VON ENDOTHELZELLEN AUS PLURIPOTENTEN STAMMZELLEN
METHOD FOR OBTAINING ENDOTHELIAL CELLS FROM PLURIPOTENT STEM CELLS

(43) Date de publication de la demande: 08.09.2021
(73) Titulaire: Adhara, 75116 Paris (FR); Centre d'Etude des Cellules Souches (CECS), 91100 Corbeil Essonnes (FR); Universite d'Evry Val d'Essonne, 91000 Evry (FR)
(72) Inventeur: BALDESCHI, Christine, 91360 Villemoisson Sur Orge (FR); DARLE, Anabelle, 77220 Favieres (FR)
(74) Mandataire: Plasseraud IP

(56) Documents cités:
- CN-A- 109 797 132
- US-A1- 2016 186 135
- MAKOTO SAHARA ET AL: "Manipulation of a VEGF-Notch signaling circuit drives formation of functional vascular endothelial progenitors from human pluripotent stem cells", CELL RESEARCH, vol. 24, no. 7, 9 May 2014 (2014-05-09), pages 820 - 841, XP055200504, ISSN: 1001-0602, DOI: 10.1038/cr.2014.59
- LIU XIAOPENG ET AL: "Differentiation of functional endothelial cells from human induced pluripotent stem cells: A novel, highly efficient and cost effective method", DIFFERENTIATION, SPRINGER VERLAG, DE, vol. 92, no. 4, 4 June 2016 (2016-06-04), pages 225 - 236, XP029789004, ISSN: 0301-4681, DOI: 10.1016/J.DIFF.2016.05.004
- HANNAH K. WILSON ET AL: "Concise Review: Tissue-Specific Microvascular Endothelial Cells Derived From Human Pluripotent Stem Cells : Stem Cell-Derived Tissue-Specific Endothelial Cells", STEM CELLS (MIAMISBURG), vol. 32, no. 12, 26 November 2014 (2014-11-26), pages 3037 - 3045, XP055382973, ISSN: 1066-5099, DOI: 10.1002/stem.1797
- GOPU SRIRAM ET AL: "Efficient differentiation of human embryonic stem cells to arterial and venous endothelial cells under feeder- and serum-free conditions", STEM CELL RESEARCH & THERAPY, vol. 6, no. 1, 1 December 2015 (2015-12-01), XP055630854, DOI: 10.1186/s13287-015-0260-5
- XIAOJUN LIAN ET AL: "Efficient Differentiation of Human Pluripotent Stem Cells to Endothelial Progenitors via Small-Molecule Activation of WNT Signaling", STEM CELL REPORTS, vol. 3, no. 5, 1 November 2014 (2014-11-01), United States, pages 804 - 816, XP055246300, ISSN: 2213-6711, DOI: 10.1016/j.stemcr.2014.09.005
- CHRISTOPH PATSCH ET AL: "Generation of vascular endothelial and smooth muscle cells from human pluripotent stem cells", NATURE CELL BIOLOGY, vol. 17, no. 8, 27 July 2015 (2015-07-27), London, pages 994 - 1003, XP055547623, ISSN: 1465-7392, DOI: 10.1038/ncb3205

## Description

La présente invention concerne un procédé d'obtention de cellules endothéliales à partir de cellules souches pluripotentes (Pluripotent Stem Cell ou PSC) embryonnaires ou de cellules souches induites à la pluripotence (iPS).

### Contexte de l'invention

Les cellules souches pluripotentes humaines, qu'elles soient embryonnaires ou induites à la pluripotence présentent une capacité à proliférer à l'identique (chaque cellule-mère donnant naissance à deux cellules-filles identiques à la première) à l'infini, sans jamais entrer en sénescence comme le font toutes les autres cellules de l'organisme, et la capacité, dans d'autres conditions de culture, à se différencier pour donner naissance à n'importe quelle cellule de l'organisme (ectoderme, endoderme et mésoderme).

Les cellules souches sont importantes en médecine régénérative (source d'intérêt majeur et prometteur pour réussir à fabriquer des organes), pour la modélisation, notamment de maladies, et le criblage pharmacologique.

Les cellules souches pluripotentes apparaissent comme une alternative possible du fait de leur propriété de prolifération illimitée et leur capacité de différenciation permettant d'obtenir à partir d'un même donneur, tous les types cellulaires d'intérêt en grande quantité. De plus, les cellules souches pluripotentes permettent d'obtenir une population homogène de cellules différenciées, contrairement aux cultures primaires (hétérogénéité, limite en nombre, risque de sénescence).

La présente invention vise une méthode d'obtention de cellules endothéliales, dans des conditions dites « cliniques », c'est-à-dire des conditions où les produits utilisés sont fabriqués selon les Bonnes Pratiques de Fabrication (BPF ou GMP - Good Manufacturing Practices) et peuvent donc être utilisés dans des études cliniques contrairement aux produits de grade « recherche » qui ne peuvent être utilisés que pour la recherche.

Les normes « BPF » constituent une notion d'assurance qualité établie par la commission européenne (ou américaine) dans le cadre de la fabrication de médicaments à usage humain ou vétérinaire (EUDRALEX en France ou FDA aux Etats-Unis). Elles ont été créées pour limiter les risques de contamination croisée des produits, en insistant sur les pratiques d'hygiène, ainsi que les risques de confusion : étiquetage/identification.

Les principes des BPF imposent l'écriture de modes opératoires et d'instructions permettant des productions de qualité régulière avec une traçabilité conforme. Ils intègrent également les procédés, la qualité du produit et la sécurité du personnel. Les BPF sont actuellement organisées en 3 parties :
- Les bonnes pratiques de fabrication des médicaments à usage humain.
- Les bonnes pratiques de fabrication pour les substances actives utilisées comme matière première dans les médicaments.
- Les documents relatifs aux bonnes pratiques de fabrication donnant les recommandations sur les exigences internationales pour la certification de lots.

Pour établir des protocoles d'obtention de cellules endothéliales « cliniques », les matières premières doivent répondre aux mêmes normes BPF. Les produits utilisés sont donc entièrement définis au niveau de leurs compositions, de leurs concentrations, de leurs origines et de leurs niveaux de stérilité.

A ce jour, il n'existe pas de moyen d'obtenir des cellules endothéliales en grande quantité selon les normes BPF. Or, les besoins sont de plus en plus nombreux, dans divers domaines d'application clinique, tels que l'obtention de tissus reconstitués utilisables en thérapie.

Les cellules HUVEC (Human Umbilical Vein Endothelial Cells) sont des cellules endothéliales issues de sang de cordon ombilical humain. Elles peuvent être obtenues en grande quantité. Toutefois, elles peuvent présenter des problèmes d'hétérogénéité et il existe le risque que les lots présentent une variabilité selon les donneurs.

### Les documents :

- Makoto Sahara et al., Cell Research, vol 24, no7, 2014, p820 841 ;
- Liu Xiaopeng et aL, Differentiation, vol 92, 2006, p 225-236;
- Hannah K. Wilson et al., Stem Cells, vol 32, 2014, p3037-3045;
- Gopu Sriram et al., Stem Cell Research & Therapy, vol 6, 2015 ;
- US2016/086135A1
- CN109797132A
   et
- Xiaojun Lian et al., Stem Cell Reports, vol 3, 2014 p804-816
décrivent différents protocoles différentiation de cellules pluripotentes humaines en cellules endothéliales.

Les cellules souches pluripotentes apparaissent comme une alternative possible du fait de leur propriété de prolifération illimitée et leur capacité de différenciation permettant d'obtenir à partir d'un même donneur, tous les types cellulaires d'intérêt en grande quantité.

A ce jour, il n'existe aucun procédé de différenciation en cellules endothéliales à partir de cellules souches humaines pluripotentes répondant aux normes BPF.

### Exposé de l'invention

La présente invention concerne un procédé d'obtention de cellules endothéliales à partir de cellules souches pluripotentes humaines d'origine embryonnaire ou cellules souches pluripotentes humaines induites qui répond aux normes BPF.

Plus particulièrement, la présente invention concerne un procédé de différenciation de cellules endothéliales à partir de cellules souches pluripotentes humaines, caractérisé en ce que:
a) à J0, les cellules souches pluripotentes humaines sont dissociées, ensemencées à une densité de 40000 à 60000 cellules/cm², de préférence environ 50000 cellules/cm², sur une matrice et cultivées en présence d'un milieu adapté à la culture des cellules pluripotentes, comprenant en outre du facteur de croissance fibroblastique 2 (« Fibroblast Growth Factor 2 » ou FGF2) et un inhibiteur de ROCK,
b) à J1, le milieu est remplacé par un milieu adapté à l'induction du mésoderme comprenant en outre un inhibiteur de GSK3 (Glycogen Synthase Kinase 3) et de la BMP4 (Bone Morphogenetic Protein 4) ;
c) à J4, le milieu est remplacé par un milieu adapté à la culture des cellules endothéliales comprenant en outre du VEGF (Vascular Endothelial Growth Factor) et de la forskoline ;
d) à J6, les cellules sont dissociées et sélectionnées pour l'expression du marqueur CD144.

Avantageusement, tous les milieux de culture et agents utilisés sont chimiquement définis et ne contiennent aucun additif d'origine animale non contrôlé aux normes de Bonnes Pratiques de Fabrication (ou BPF).

Par « cellules souches pluripotentes » on entend toute cellule indifférenciée, capable de s'auto-renouveler à l'infini, de se différencier en tous types cellulaires (ectoderme, endoderme, mésoderme). De manière préférée, les cellules souches pluripotentes selon l'invention sont des cellules souches pluripotentes humaines (« human pluripotent stem cell » ou « hPSC »).

Dans un mode de réalisation, les cellules souches pluripotentes sont des cellules souches embryonnaires humaines (« human embryonic stem cell » ou « hESC »). Il existe de nombreuses lignées de hESC, dont la lignée RC-9 (« Roslin Cells 9 »), qui a été développée en tant que lignée hESC de grade clinique, citée comme lignée cellulaire de référence.

Les cellules souches pluripotentes humaines sont obtenues par des méthodes qui ne nécessitent pas la destruction d'embryons.

Dans un autre mode de réalisation, les cellules souches sont des cellules souches humaines induites à la pluripotence (« human induced pluripotent stem cell » ou « hiPSC »). Il existe de nombreuses lignées de hiPSC commerciales ou fabriquées selon les différentes techniques de reprogrammation existantes (épisome, ARNm, vecteur viral Sendaï).

Selon un mode de réalisation de l'invention, les cellules souches pluripotentes sont des cellules hiPSC spécifiques d'un donneur, obtenues par reprogrammation de cellules mononucléaires de sang périphérique dudit donneur. Les protocoles de reprogrammation de cellules mononucléaires de sang périphérique du donneur ou de cellules CD34+ isolées de sang de cordon ombilical sont connus de l'homme de l'art.

Par « matrice » ou « coating », on entend tout substrat permettant la culture des cellules souches en monocouche. De manière préférée, la matrice utilisée dans le procédé selon l'invention est une matrice protéique définie. De manière préférée, la matrice est choisie dans le groupe constitué par Matrigel^{™}, L7 coating^{™}, la laminine et la vitronectine. De manière particulièrement préféré, la matrice est la matrice L7^{™} commercialisée par la société Lonza sous la référence FP-5020.

Par « milieu adapté à la culture des cellules pluripotentes » on entend tout milieu qui contient les nutriments et facteurs permettant la culture *in vitro* de cellules pluripotentes.

De manière préférée, le milieu adapté à la culture des cellules pluripotentes est choisi parmi le milieu iPS Brew XF GMP commercialisé par la société Miltenyi Biotec et le milieu iPS Stempro commercialisé par la société ThermoFisher.

Lors de l'étape a), le milieu est supplémenté de « facteur de croissance fibroblastique 2 » ou « FGF2 » ou « Fibroblast Growth Factor 2 ». Typiquement, le FGF2 est utilisé à une concentration finale de 5 à 20 ng/ml, de préférence environ 10 ng/ml.

Lors de l'étape a), le milieu est supplémenté d'un inhibiteur de ROCK. De manière préférée, l'inhibiteur de ROCK est fourni au sein du supplément Revitacell commercialisé par la société Gibco.

Par « milieu adapté à l'induction du mésoderme » on entend tout milieu qui contient les nutriments et facteurs permettant l'induction de la voie mésodermique des cellules pluripotentes. De manière préférée, le milieu adapté à l'induction du mésoderme comprend les suppléments N2 et B27. Le milieu N2B27 est un mélange 1 :1 de milieu KO DMEM-F12 CTS et du milieu Neurobasal CTS, supplémenté avec du Glutamax CTS, du N2 CTS et du B27 CTS.

Lors de l'étape b), le milieu adapté à l'induction du mésoderme comprend également un inhibiteur de GSK3 et de la BMP4.

Après l'étape c), à J5 le milieu est à nouveau remplacé par un milieu adapté à la culture des cellules endothéliales comprenant en outre du VEGF (Vascular Endothelial Growth Factor) et de la forskoline ;

L'homme de l'art a, à sa disposition, un certain nombre d'agents connus pour inhiber la kinase GSK3. Typiquement, l'inhibiteur de GSK3 peut être le Chir99021 commercialisé par la société Tocris.

Typiquement, la concentration finale en Chir99021 est comprise entre 5 et 10 µM, de préférence environ 6 µM.

Typiquement, la concentration finale en BMP4 est comprise entre 15 et 50 ng/ml, de préférence environ 25 ng/ml.

Par « milieu adapté à la culture des cellules endothéliales » on entend tout milieu qui contient les nutriments et facteurs permettant la culture *in vitro* de cellules endothéliales.

De manière préférée, le milieu adapté à la culture des cellules endothéliales est le milieu CnT-Endo commercialisé par la société CellnTec.

Lors de l'étape c), le milieu est supplémenté de VEGF et de forskoline.

De manière préférée, le VEGF est fourni à une concentration finale comprise entre 100 et 300 ng/ml, de manière encore plus préférée environ 200 ng/ml.

De manière préférée, la forskoline est fournie à concentration finale comprise entre 1 et 3 µM, de manière encore plus préférée environ 2 µM.

Selon un mode de réalisation de l'invention, le milieu est renouvelé à J5.

A J6, une certaine partie de la population de cellules s'est différenciée en cellules endothéliales.

Afin d'enrichir la population en cellules endothéliales, une étape de sélection est réalisée à l'étape d). Cette sélection peut se faire par tri cellulaire.

Selon un mode de réalisation, la sélection est réalisée par cytométrie en flux.

Selon un autre mode de réalisation, la sélection est réalisée à l'aide de billes magnétiques recouvertes d'un anticorps spécifique du marqueur des cellules endothéliales tels que CD34+ ou CD144 ou CD31 +, de préférence CD144.

La présente invention concerne également une population de cellules endothéliales directement obtenue par le procédé décrit ci-dessus. De manière avantageuse, la population de cellules endothéliales est homogène, c'est-à-dire que plus de 90%, de manière préférée plus de 95%, plus de 98%, ou plus de 99% des cellules sont positives pour le marqueur CD144.

La présente invention concerne également l'utilisation de la population de cellules endothéliales pour la fabrication d'un tissu, de préférence un tissu dermique ou substitut de peau.

### Brève description des Figures

D'autres caractéristiques, détails et avantages de l'invention apparaîtront à la lecture des Figures annexées.
**Fig. 1**

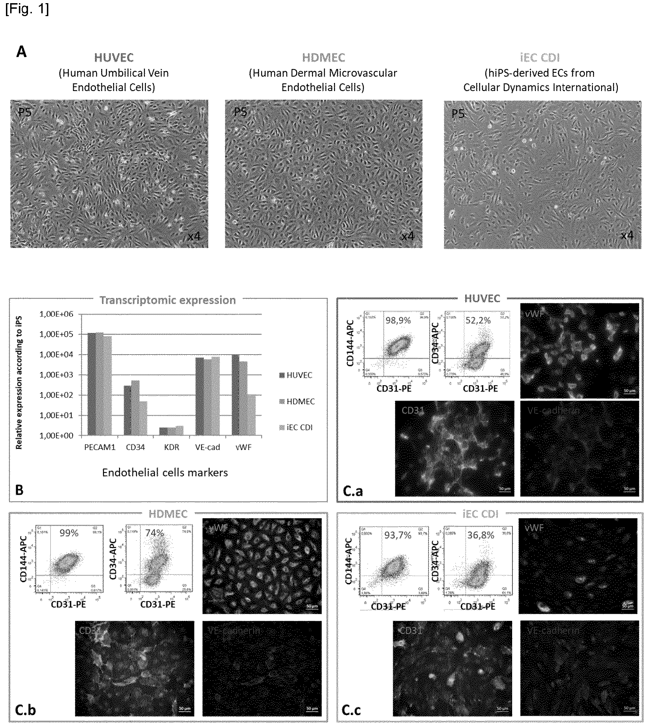

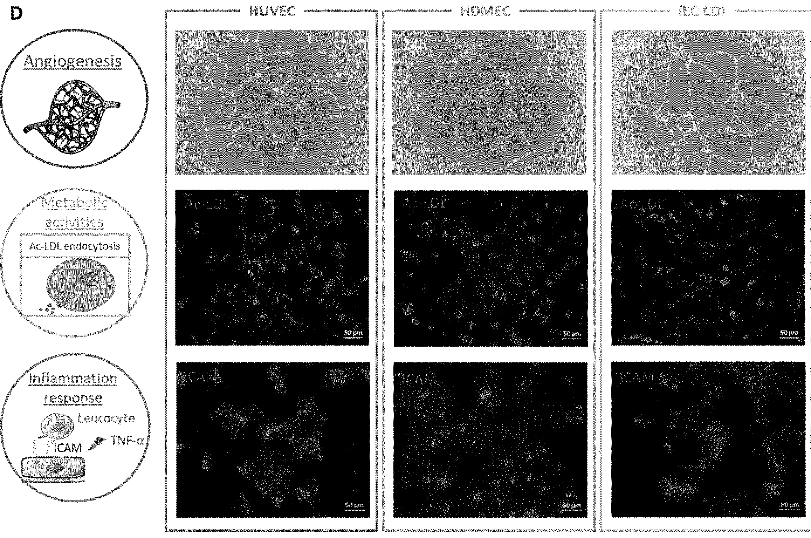

   [Fig. 1] représente la caractérisation des cellules endothéliales de référence à P5.
   (A) Morphologie des cellules endothéliales de référence HUVEC, HDMEC et iEC CDI. (B) Profil transcriptomique des cellules de référence, analyse par qPCR des marqueurs spécifiques des cellules endothéliales PECAM1 (ou CD31), CD34, KDR, VE-cadherin (ou CD144) et vWF. (C) Profil protéique, analyse par FACS de CD31/CD34 et CD31/CD144 et par immunofluorescence de CD31, VE-cadherin (ou CD144) et vWF. a) HUVEC b) HDMEC c) iEC CDI (D) Fonctionnalité : formation de tubules sur GFR-matrigel, endocytose d'Ac-LDL, réponse à l'inflammation par traitement au TNFα et observation de l'expression d'ICAM.
**Fig. 2**

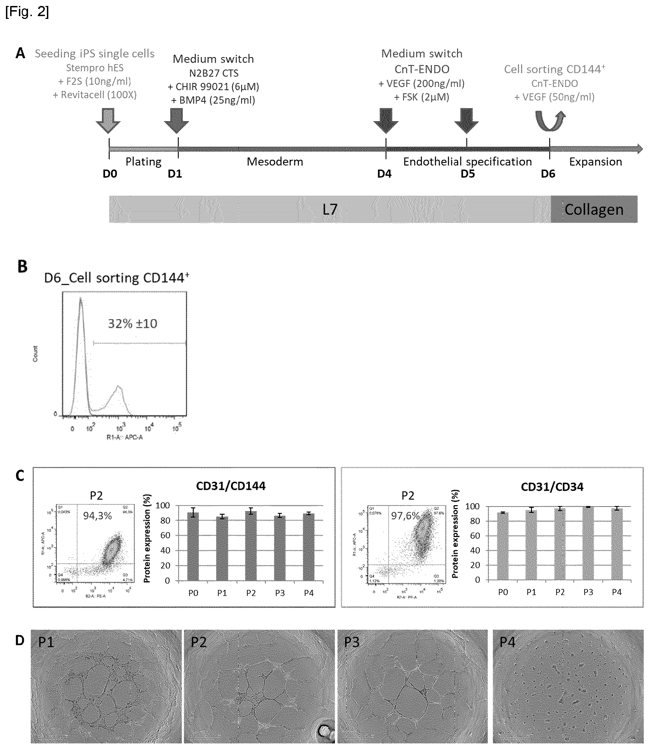

   [Fig. 2] représente le procédé selon l'invention et la caractérisation des cellules endothéliales obtenues.
   (A) Protocole clinique validé. (B) FACS CD144-APC à J6 (avant sélection). (C) FACS au cours des passages pour l'analyse des co-marquages CD31/CD144 et CD31/CD34 avec les résultats à P2 représentatifs des différents passages. (D) Fonctionnalité des cellules produites au cours des passages et formation de tubules sur GFR-matrigel.

### Exemple 1 : Caractérisation des cellules endothéliales de référence

Des cellules endothéliales primaires HUVEC (Human Umbilical Vein Endothelial Cells) et HDMEC (Human Dermal Microvascular Endothelial Cells) ainsi que des cellules endothéliales dérivées d'iPS produites par Cellular Dynamics International (iEC CDI) ont été utilisées comme cellules de référence pour réaliser l'ensemble de la mise en place et la validation des contrôles qualité des cellules endothéliales dérivées d'iPS : profil transcriptomique (qPCR), profil protéique (FACS, immunofluorescence) et fonctionnalités (formation de tubules sur matrigel, réponse à l'inflammation, capacité d'endocytose).

Les marqueurs analysés pour caractériser les cellules endothéliales sont CD31 codé par le gène PECAM1 et CD144 codée par le gène VE-Cadherin, deux protéines membranaires impliquées dans les jonctions intercellulaires entre cellules endothéliales et qui permettent l'intégrité de l'endothélium. Le marqueur cytoplasmique vWF est impliqué dans le recrutement des plaquettes lors d'une lésion des vaisseaux et est présent chez les cellules endothéliales matures. Il permet donc d'avoir une idée sur la maturité des cellules. La protéine CD34 est un marqueur commun aux cellules endothéliales et hématopoïétiques car elle est exprimée à partir du stade hémangioblaste, précurseur des deux types cellulaires. Le dernier marqueur analysé est VEGFR2 codé par KDR, récepteur du VEGF qui lui-même est impliqué dans la stimulation de l'angiogenèse et la survie des cellules endothéliales.

La figure 1A montre que les 3 types de cellules de référence présentent la même morphologie. Elles ont un profil transcriptomique similaire sauf pour le marqueur vWF qui montre une maturité plus faible des iEC CDI par rapport aux HUVEC et HDMEC (Figure 1). Les HUVEC et HDMEC expriment à plus de 98% les marqueurs membranaires CD31 et CD144. Les iEC CDI les expriment à 93,7%. Quant au marqueur CD34, on observe pour les 3 types cellulaires une double population, une positive et une négative à environ 50%. L'immunofluorescence permet de vérifier que le marqueur de maturité vWF est bien exprimé dans le cytoplasme des cellules (Figure 1C a, b et c).

Pour vérifier la fonctionnalité des cellules, leur capacité à s'organiser en structure « tubule-like » est testée sur matrigel durant 24h. Les 3 lignées de cellules contrôles forment des structures en réseau (Figure 1D).

L'activité métabolique des cellules endothéliales est testée grâce au test d'endocytose d'Ac-LDL couplé à un fluorochrome. Celui-ci est ajouté au milieu de culture des cellules pendant 4h puis les cellules sont rincées. L'Ac-LDL endocyté reste dans les cellules et peut être observé par la présence de fluorescence à l'intérieur des cellules démontrant leur capacité d'endocytose (Figure 1D).

Le dernier test de fonctionnalité des cellules endothéliales a pour but de montrer leur capacité à répondre à l'inflammation via la stimulation au TNFα pendant 24h. Ce traitement permet la surexpression d'ICAM, un marqueur de l'inflammation (stimulateur de l'adhésion et transmigration des leucocytes au travers de l'épithélium endothélial). Sans traitement, on n'observe aucune expression d'ICAM dans les cellules (non montré) alors qu'avec traitement les cellules expriment ICAM et de façon similaire entre les 3 types cellulaires (Figure 1D).

Ces résultats montrent que les cellules de référence semblent fonctionnelles et expriment les marqueurs spécifiques des cellules endothéliales. Ce sont donc de bons contrôles pour les cellules issues des différenciations à partir de cellules souches pluripotentes.

### Exemple 2 : Obtention de cellules endothéliales dérivées de hPSC et caractérisation phénotypique desdites cellules

Les cellules hiPSC sont dissociées à l'accutase puis ensemencées à 50000c/cm² sur du coating L7 en milieu Stempro hESC SFM supplémenté de 10ng/ml de FGF2 et de Revitacell au 1/100. Le lendemain, à J1 le milieu est remplacé par du milieu d'induction au mésoderme : milieu N2B27 CTS (mélange 1 :1 de KO DMEM-F12 et du milieu Neurobasal CTS supplémenté avec du Glutamax CTS, du N2 CTS et du B27) avec du Chir99021 à 6uM et de la BMP4 à 25ng/ml.

A J4, le milieu est remplacé par le milieu de spécification endothéliale : milieu CnT-ENDO supplémenté avec 200ng/ml de VEGF et 2µM de forskoline. Le milieu est renouvelé le lendemain. Au 6ème jour de différenciation, les cellules différenciées sont dissociées à l'accutase et sélectionnées en utilisant des billes magnétiques CD144+ avec des colonnes LS commercialisées par Miltenyi Biotec. Les cellules positives, c'est-à-dire les cellules endothéliales différenciées, sont collectées puis congelées en cryostor.

Les cellules sont ensuite décongelées et ensemencées sur une matrice de collagène I à 10µg/ml à 20000c/cm² en milieu CnT-ENDO supplémenté avec 50ng/ml de VEGF. Le milieu est changé tous les 2 jours et les cellules sont passées tous les 3-4 jours.

L'ensemble de ces étapes est schématisé dans la Figure 2A.

Les cellules ainsi obtenues par ce procédé ont été caractérisées du point de vue phénotypique et fonctionnel. Les cellules obtenues par le procédé de l'invention expriment bien les marqueurs CD31, CD144 et CD34 (Figure 2C) et sont fonctionnelles jusqu' à p3 (Figure 2D).

### Exemple 3 : Comparaison de différents milieux de culture et de différents coatinas

Le protocole de l'exemple 2 a été réalisé en utilisant différents milieux de culture et différents coatings, comme indiqué dans le tableau 1 ci-dessous.

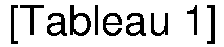

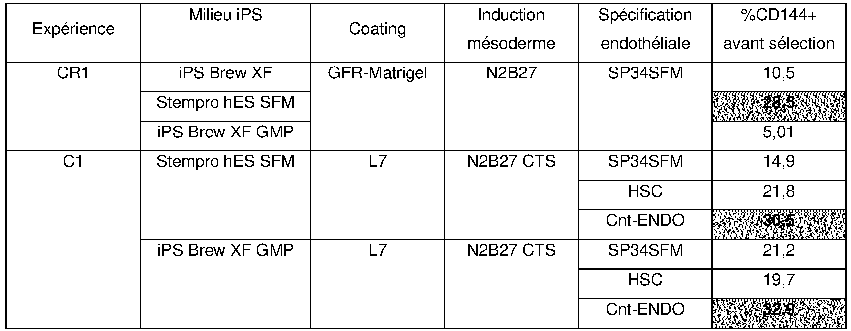

Les rendements obtenus (en % de cellules CD144-positives (ou CD144+) à l'issue de l'étape de différenciation) ont été comparés.

Il résulte de ces tests que les milieux Stempro hES SFM et iPS Brew XF GMP sont tous les deux efficaces comme milieu de culture des cellules pluripotentes.

Le coating L7, de grade clinique, est aussi efficace que le coating GFR-matrigel.

Pour l'étape d'induction du mésoderme, le milieu CnT-ENDO est plus efficace que les autres milieux testés.

## Revendications

1. Procédé de différenciation en cellules endothéliales à partir de cellules souches pluripotentes **caractérisé en ce que**:
a) à J0, les cellules souches pluripotentes sont dissociées, ensemencées à une densité de 40000 à 60000 cellules/cm², de préférence environ 50000 cellules/cm², sur une matrice et cultivées en présence d'un milieu adapté à la culture des cellules pluripotentes, comprenant en outre du facteur de croissance fibroblastique 2 (« Fibroblast Growth Factor 2 » ou FGF2) et un inhibiteur de ROCK ;
b) à J1, le milieu est remplacé par un milieu adapté à l'induction du mésoderme comprenant en outre un inhibiteur de GSK3 (Glycogen Synthase Kinase 3) et de la BMP4 (Bone Morphogenetic Protein 4) ;
c) à J4, le milieu est remplacé par un milieu adapté à la culture des cellules endothéliales comprenant en outre du VEGF (Vascular Endothelial Growth Factor) et de la forskoline ;
d) à J6, les cellules sont dissociées et sélectionnées pour l'expression du marqueur CD144, dans lequel ledit procédé répond aux normes de Bonnes Pratiques de Fabrication (BPF).

2. Procédé selon la revendication 1 **caractérisé en ce que** le FGF2 est utilisé à une concentration de 5 à 20 ng/ml, de préférence environ 10 ng/ml, à l'étape a).

3. Procédé selon l'une quelconque des revendications précédentes, **caractérisé en ce que** l'inhibiteur de GSK3 est le Chir99021, de préférence utilisé à une concentration 5 à 10 µM, de manière encore plus préférée, à une concentration d'environ 6 µM, à l'étape b)

4. Procédé selon l'une quelconque des revendications précédentes, **caractérisé en ce que** le BMP4 est utilisé à une concentration comprise entre 15 et 50 ng/ml, de préférence environ 25 ng/ml à l'étape b).

5. Procédé selon l'une quelconque des revendications précédentes, **caractérisé en ce que** le VEGF est utilisé à une concentration comprise entre 100 et 300 ng/ml, de préférence environ 200 ng/ml à l'étape c).

6. Procédé selon l'une quelconque des revendications précédentes, **caractérisé en ce que** la forskoline est utilisée à une concentration comprise entre 1 et 3 µM, de préférence environ 2 µM à l'étape c).

7. Procédé selon l'une quelconque des revendications précédentes, **caractérisé en ce que** les cellules souches pluripotentes sont des cellules souches humaines induites à la pluripotence.

## Patentansprüche

1. Verfahren zur Differenzierung in Endothelzellen aus pluripotenten Stammzellen, **dadurch gekennzeichnet, dass**:
a) an Tag 0 die pluripotenten Stammzellen dissoziiert werden, mit einer Dichte von 40.000 bis 60.000 Zellen/cm², bevorzugt etwa 50.000 Zellen/cm², auf eine Matrix ausgesät werden und in Gegenwart eines für die Kultivierung von pluripotenten Zellen geeigneten Mediums kultiviert werden, ferner umfassend Fibroblasten-Wachstumsfaktor 2 (<< Fibroblast Growth Factor 2 >> oder FGF2) und einen ROCK-Inhibitor;
b) an Tag 1 das Medium durch ein zur Induktion des Mesoderms geeignetes Medium ersetzt wird, das ferner einen GSK3-Inhibitor (Glycogen Synthase Kinase 3) und BMP4 (Bone Morphogenetic Protein 4) umfasst;
c) an Tag 4 das Medium durch ein für die Endothelzellkultur geeignetes Medium ersetzt wird, das ferner VEGF (Vascular Endothelial Growth Factor) und Forskolin enthält;
d) an Tag 6 die Zellen dissoziiert werden und für die Expression des Markers CD144 selektioniert werden, wobei das Verfahren den Standards der Guten Herstellungspraxis (GMP) entspricht.

2. Verfahren nach Anspruch 1, **dadurch gekennzeichnet, dass** in Schritt a) FGF2 in einer Konzentration von 5 bis 20 ng/ml, bevorzugt etwa 10 ng/ml, verwendet wird.

3. Verfahren nach einem der vorhergehenden Ansprüche, **dadurch gekennzeichnet, dass** in Schritt b) der GSK3-Inhibitor Chir99021 ist, bevorzugt in einer Konzentration von 5 bis 10 µm, stärker bevorzugt in einer Konzentration von etwa 6 µm.

4. Verfahren nach einem der vorhergehenden Ansprüche, **dadurch gekennzeichnet, dass** in Schritt b) BMP4 in einer Konzentration zwischen 15 und 50 ng/ml, bevorzugt etwa 25 ng/ml, verwendet wird.

5. Verfahren nach einem der vorhergehenden Ansprüche, **dadurch gekennzeichnet, dass** in Schritt c) VEGF in einer Konzentration zwischen 100 und 300 ng/ml, bevorzugt etwa 200 ng/ml, verwendet wird.

6. Verfahren nach einem der vorhergehenden Ansprüche, **dadurch gekennzeichnet, dass** in Schritt c) Forskolin in einer Konzentration zwischen 1 und 3 µm, bevorzugt etwa 2 µm, verwendet wird.

7. Verfahren nach einem der vorhergehenden Ansprüche, **dadurch gekennzeichnet, dass** die pluripotenten Stammzellen zur Pluripotenz induzierte menschliche Stammzellen sind.

## Claims

1. A method for differentiating into endothelial cells from pluripotent stem cells, **characterized in that**:
a) on D0, the pluripotent stem cells are dissociated, seeded at a density of 40 000 to 60 000 cells/cm², preferably approximately 50 000 cells/cm², on a matrix and cultured in the presence of a medium suitable for the culture of pluripotent cells, further comprising fibroblast growth factor 2 (FGF2) and a ROCK inhibitor;
b) on D1, the medium is replaced with a medium suitable for mesoderm induction, further comprising a GSK3 (Glycogen Synthase Kinase 3) inhibitor and BMP4 (Bone Morphogenetic Protein 4);
c) on D4, the medium is replaced with a medium suitable for the culture of endothelial cells, further comprising VEGF (Vascular Endothelial Growth Factor) and forskolin;
d) on D6, the cells are dissociated and selected for expression of the CD144 marker, wherein said process meets good manufacturing practices (GMP) standards.

2. The method according to claim 1, **characterized in that** the FGF2 is used at a concentration from 5 to 20 ng/ml, preferably approximately 10 ng/ml, in step a).

3. The method according to any one of the preceding claims, **characterized in that** the GSK3 inhibitor is Chir99021, preferably used at a concentration of 5 to 10 µM, even more preferably at a concentration of approximately 6 µM, in step b).

4. The method according to any one of the preceding claims, **characterized in that** the BMP4 is used at a concentration between 15 and 50 ng/ml, preferably approximately 25 ng/ml, in step b).

5. The method according to any one of the preceding claims, **characterized in that** the VEGF is used at a concentration between 100 and 300 ng/ml, preferably approximately 200 ng/ml, in step c).

6. The method according to any one of the preceding claims, **characterized in that** the forskolin is used at a concentration between 1 and 3 µM, preferably approximately 2 µM in step c).

7. The method according to any one of the preceding claims, **characterized in that** the pluripotent stem cells are human induced pluripotent stem cells.
